# EUROPEAN PATENT APPLICATION

(11) **EP 4 843 617 A1**
(43) Date of publication of application: **25.11.2026**
(21) Application number: 25178316.3
(22) Date of filing: 22.05.2025
(51) Int. Cl.: A61N 5/10

(54) **GENERATING A HYBRID RADIATION TREATMENT PLAN FOR PROTON THERAPY**

(71) Applicant: Siemens Healthineers International AG, 6312 Steinhausen (CH)
(72) Inventor: HYTONEN, Roni, 02130 Espoo (FI); VANDERSTRAETEN, Reynald, 1170 Uccle (BE); VERBAKEL, Willem, 1065HR Amsterdam (NL)
(74) Representative: Müller Schupfner & Partner Patent- und Rechtsanwaltspartnerschaft mbB (Muc)

(57) **Abstract**

The invention relates to a computer-implemented method for generating a hybrid radiation treatment plan for proton therapy of a patient, the method comprising: (a) receiving a patient anatomy including a target volume (1); (b) defining at least one conventional radiation field (2) comprising one or more Bragg peak proton beams, each Bragg peak proton beam having a Bragg peak within the target volume; (c) defining at least one transmission radiation field (4) consisting of one or more transmission proton beams, each transmission proton beam having sufficiently high energy to reach a distal exit side of the patient; and (d) providing a hybrid radiation treatment plan comprising the at least one conventional radiation field (2) and the a least one transmission radiation field (4).

## Description

The invention relates to a computer-implemented method for generating a hybrid radiation treatment plan for proton therapy of a patient, to a computer program, a digital storage medium, a system for generating a hybrid radiation treatment plan and to a proton therapy system.

Independent of the grammatical term usage, individuals with male, female or other gender identities are included within the term.

In proton therapy planning, one or more proton fields are generally used to cover the target volume with radiation. Traditionally, each field consists of multiple energy layers, each delivering the bulk of radiation to a specific depth within the patient, by virtue of an effect of proton beams called the "Bragg peak". The advantage of this approach is a highly conformal dose distribution that does not produce an exit dose, as the beam terminates at the Bragg peak within the target volume.

However, the use of Bragg peak beams in proton treatment is not fully without drawbacks: the lower energies cause beams with larger penumbras, i.e. the dose fall-off to the sides is not very steep, which again can increase dose to nearby organs at risk (OARs). Also, the Bragg peak needs to be very precisely positioned, making the approach susceptible to errors because of density variations. Furthermore, it takes a significant amount of time to modulate the energy of the proton beam, i.e. to switch from one energy layer to the next, and to scan the beam through each of the layers. It is imperative for the patient to remain motionless during this time to ensure sufficient dose coverage of the target volume. In cases where the beam is gated by a patient's breath hold, the delivery is abrupted whenever the patient runs out of breath. Longer delivery times require more breath holds which can become difficult for a patient, which can further prolong the delivery. In addition to patient comfort and patient's ability to remain motionless, the prolonged treatment times can also affect the number of patients a proton facility is able treat.

Due to the uncertainties in proton beam delivery and to account for density uncertainties and changes in a patient, some hospitals prefer to treat patients with combined proton and photon therapy. However, this leads to increased normal tissue dose, and of course generally increases the duration and complexity of the treatment for both patient and hospitals.

A non-conventional way of proton irradiation is the use of so-called transmission beams that deliver the Bragg peak behind the patient and therefore have a relatively constant dose vs. depth profile along the beamline. This requires high energy beams, which have a smaller penumbra. Lack of energy switching makes the delivery of transmission beams very fast. The disadvantage is that the patient does not benefit from the stopping of the beam in the target at the Bragg peal. Furthermore, the constant dose reduces the number of optimization possibilities. This makes it more difficult to fulfil the dosimetric requirements for the target, in particular achieving a homogeneous dose across the target tissue.

In "Ultra-High Dose Rate Transmission Beam Proton Therapy for Conventionally Fractionated Head and Neck Cancer: Treatment Planning and Dose Rate Distributions", Cancers 2021, 13, 1859, P. van Marlen. M. Dahele, M. Folkerts et al. investigated using only transmission beams for the treatment of head and neck cancer. It was found that the plan quality was better than for photon VMAT (Volumetric Modulated Arc Therapy), but could not compete with the traditional Bragg peak IMPT (Intensity Modulated Proton Therapy).Thus, in this study, transmission beams could not be identified as a preferable treatment option.

W. Kong, M. Huiskes et al. in "Reducing the lateral dose penumbra in IMPT by incorporating transmission pencil beams" in Radiotherapy and Oncology 198 (2024) 110388 allowed incorporating transmission pencil beams in every radiation field, i.e. used a combination of transmission beams and Bragg peak beams for every radiation direction. While this approach improved plan quality compared to traditional IMPT, it required very long calculation times and cannot solve the problem of long delivery times.

It is therefore an object of the invention to reduce the delivery times for proton therapy, while still achieving a good plan quality, in particular a plan quality comparable to IMPT (Intensity Modulated Proton Therapy).

This object is achieved by a method of claim 1, a computer product and digital storage medium of claim 11, a system of claim 14 and a proton therapy system of claim 15. Preferred embodiments are given in the dependent claim.

According to a first aspect, the invention is directed to a computer-implemented method for generating a hybrid radiation treatment plan for proton therapy of a patient, the method comprising:
(a) receiving a patient anatomy including a target volume, the target volume to be treated with a proton therapy;
(b) defining at least one conventional radiation field comprising one or more Bragg peak proton beams, each Bragg peak proton beam having a Bragg peak within the target volume;
(c) defining at least one transmission radiation field consisting of one or more transmission proton beams, each transmission proton beam having sufficiently high energy to reach a distal exit side of the patient; and
(d) providing a hybrid radiation treatment plan comprising the at least one conventional radiation field and the at least one transmission radiation field.

The invention thus proposes a novel method and system for generating and optimizing hybrid radiation treatment plans. In particular, a hybrid radiation treatment plan will contain two different kinds of proton treatment fields:
- at least one conventional radiation field or "conventional field", also referred to as Bragg peak field or as "traditional field", having multiple energy layers spanning the target structure, and
- at least one transmission radiation field or "transmission field", having a single high-energy layer covering the entire target structure.

The advantage of the invention is that the introduction of one or more transmission fields drastically reduces the delivery time and improves robustness, while maintaining the dosimetric quality of the plan. In addition to significantly reduced delivery times, hybrid plans are inherently robust against density or patient positioning errors, as their dose profile resembles that of a photon beam. However, the proton transmission beam's shape can be altered more freely than that of a photon beam, and the lateral dose fall-off is sharper, also sharper than for a Bragg-peak beam, reducing dose to healthy tissue, while achieving a comparable dose coverage. Therefore, the invention also removes the need for treating patients with combined proton and photon therapy. Moreover, the short delivery time of the transmission fields allow for a larger flexibility for switching treatments between multiple proton treatment rooms connected to the same cyclotron. When several tens of seconds are needed for delivery of each radiation field, switching of treatments between rooms will often be done after delivery of all radiation fields. When using a treatment plan generated according to the invention, the delivery of a transmission fields takes only a few seconds. Therefore, switching can also be done in between treatment fields. Using transmission fields requires no hardware changes, and they are DICOM compliant.

A "hybrid radiation treatment plan" is herein also referred to as "hybrid plan" and describes a proton treatment plan comprising at least one conventional field and at least one transmission field. A treatment plan defines all relevant parameters of the radiation fields, so that it is ready to be delivered to the patient when he or she comes in for the treatment.

In this disclosure, the term "radiation field" is to be understood broadly. It may refer to one or a plurality of proton beams delivered substantially in one direction, or at one gantry rotation angle. A radiation field may be comprised of a single-energy proton beam, but in the case of conventional radiation fields, may also comprise multiple beams having multiple energies or multiple energy layers. A "radiation field" is generally delivered in between Gantry rotations. In IMPT, a radiation field often comprises a number of so-called pencil beams, which are delivered sequentially each having a specific energy. This process is also referred to as pencil-beam scanning (PBS) delivery.

A "conventional radiation field" is a radiation field having at least one Bragg peak beam. Generally, it will have several Bragg peak beams or energy layershaving different energies in order to cover the entire target volume. When a "conventional radiation field" is delivered in PBS, the energy needs to be adjusted before the beam moves on to the next spot, resulting in long delivery times. A conventional radiation field may comprise individual beams, e.g. pencil beams. The energy of individual beams can vary depending on the target location, as the Bragg peak of higher-energy beams penetrates deeper in the patient tissue. Thus, the proton energies in a conventional radiation field may vary within a wide range.

A "transmission radiation field" is a radiation field comprising only transmission proton beams having sufficiently high energy to reach a distal exit side of the patient. In other words, the Bragg peak is behind the patient, so that the transmission beam does not end within the target volume, but transverses the patient anatomy. In particular, a transmission field contains no Bragg peak beam. A transmission field may also be comprised of several proton beams. However, since the proton beams may have a single energy, it is possible to deliver a transmission field in fewer or only one proton beam. The proton energy of the transmission field is typically the maximum energy that the delivery system is capable of. Preferably, it is higher than 200 MeV, more preferred higher than 220 MeV, most preferred higher than 240 MeV, for example 244 MeV.

A "conventional radiation field" may in addition comprise one or more transmission beams, in which case it is referred to as a combined radiation field. While this may give some advantages in achieving plan objectives due to the sharp penumbra of transmission beams, the inclusion of a transmission beam among a plurality of Bragg peak beams does not help to reduce the delivery times. The energy still needs to be switched in between individual pencil beams, and therefore such a combined radiation field comprising one or more Bragg peak proton beams and one or more transmission proton beams is still considered herein a "conventional radiation field". In an alternative embodiment, a "conventional radiation field" does not comprise any transmission beams.

The invention is not limited as to the method of proton beam delivery. It may be done by IMPT, or by pencil-beam scanning, but the invention may also be applied to other beam delivery modalities, such as line scanning, scattering or FLASH treatment plans.

In FLASH, radiation is delivered at a very high dose rate, which has been found to result in favourable treatment outcomes. In an embodiment, at least on of the radiation field(s), in particular one or more of the transmission radiation fields, is/are delivered at ultra-high dose rates, in particular at >40 Gy/sec. This is advantageous, because it further reduces delivery time, and may contribute to the FLASH effect originating from the ultra-fast delivery. Thus, in an embodiment the hybrid radiation treatment plan may include one or more transmission radiation fields delivered at ultra-high dose rates.

A "hybrid radiation treatment plan", also referred to as "hybrid plan" herein, is a treatment plan comprising at least one conventional radiation field and at least one transmission radiation field. The term is to be understood broadly herein. A hybrid plan generally comprises the information required to deliver the specified treatment to the patient. As is known in the art, a physician responsible for a cancer patient will specify the dose to be delivered to the tumour, as well as other plan objectives, such as a maximum dose to be delivered to nearby organs at risk. These constraints are also referred to as "plan objectives". The plan objectives are used during treatment planning to develop the treatment plan which is then implemented when the patient comes in for radiation therapy. In order to generate the radiation treatment plan, the method also requires the patient anatomy including the target volume. The patient anatomy may be provided in any form, preferably as a three-dimensional (3D) image. Often, the 3D image will be a CT-image acquired for the purpose of radiation treatment planning. The target volume is generally a structure within the patient anatomy, for example a tumour or other tissue affected by disease, which is to be treated with proton therapy. Generally, the target volume is delineated or segmented on a 3D image representing the patient anatomy.

The method comprised defining at least one conventional radiation field and at least one transmission radiation field. The term "defining" herein is to be understood broadly. The term "defining" preferably includes defining at least some of the parameters which are necessary to deliver the radiation field, such as the proton energies or energies, the number of individual proton beams and the gantry rotation angle. The step of defining covers any methods by which these parameters relating to each radiation field may be generated. In particular, they may be defined by means of user input. Alternatively or additionally, they may be defined automatically. In a preferred embodiment explained below, the transmission and radiation fields will be generated by means of an optimisation process, which takes into account at least the plan objectives, but preferably also more specific objectives for the transmission radiation field(s) and the conventional radiation field(s). In an embodiment, the process of defining may comprise a first step where a user inputs at least the type of the field (conventional or transmission) and possible the gantry angle. In a next step, the other parameters are optimised to meet some or all of the objectives.

The hybrid radiation treatment plan may comprise between 1 and 20 transmission radiation fields, more preferred between 2 and 10, for example between 2 and 6. Evidently, the number of transmission radiation fields depends on the patient anatomy and target structure to be treated. However, it has been shown that several transmission fields may be included while still having an excellent dose distribution with sharp dose fall-off around the target volume. This is a surprising finding, since the general belief so far was that conventional fields would be superior due to the Bragg peak and thus absence of radiation dose behind the target volume. However, it appears that the sharp penumbra of the transmission beams may outweigh this disadvantage. Furthermore, the transmission beams are not susceptible to density variations in the beam, and are therefore very robust, whereas for Bragg Peak beams, a small variation in density could lead to the too deep or too shallow placement of the Bragg Peak dose.

The hybrid treatment plan may also comprise 1 to 8 conventional radiation fields, more preferred 1 to 6, more preferred 1 to 3, still more preferred 1 to 2 conventional radiation fields. By limiting the number of conventional radiation fields as much as possible, the field delivery time may be significantly reduced, thereby minimizing problems connected with patient movement. By the inventive optimization algorithm, the overall plan objectives may still be achieved.

The method of the invention is generally carried out with the aid of a processing unit, as the definition of radiation fields is complex, and especially the optimisation to meet the plan objectives is computationally expensive. Therefore, it is a computer-implemented method.

According to an embodiment, the method comprises the steps of
(i) receiving a definition of at least one conventional radiation field and of at least one transmission radiation field;
(ii) receiving one or more plan objectives for the hybrid radiation treatment plan, the plan objectives comprising at least a minimum dose to be delivered within the target volume and optionally a maximum dose to be delivered to an organ at risk within the patient anatomy;
(iii) calculating at least one plan cost value of the hybrid radiation treatment plan, wherein the at least one plan cost value is a measure of how well the plan objectives are being fulfilled by the hybrid radiation treatment plan; and
(iv) minimizing the at least one plan cost value by adjusting the at least one conventional radiation field and/or the at least one transmission radiation field.

Thus, the invention is also directed to an optimizing algorithm capable of handling hybrid treatment plans.

By receiving a definition of the conventional field and radiation field, the algorithm receives the initial data of the planned radiation fields, such as the number of conventional radiation fields, transmission radiation fields, and optionally, the gantry rotation angles. The definitions may be provided by a user, based on prior experience. They may also be provided from a database of suitable example treatment plans for different patient anatomies and target volumes.

The method further comprises receiving one or more plan objectives for the hybrid radiation treatment plan. These plan objectives generally comprise at least a minimum dose to be delivered to the target volume. Optionally, the plan objectives may also include maximum doses to be delivered to one or more organs at risk within the patient anatomy. The plan objectives may generally comprise a dose distribution which is to be achieve in the patient anatomy. The plan objectives may also comprise further parameters to be achieved by the treatment plan.

In an embodiment, the plan objectives include that the delivery of the treatment plan should be as fast as possible, or below a certain threshold (in seconds or minutes). In other words, the optimizer is requested to determine the maximum contribution of the transmission beams, which have a very short delivery time, which is possible while still achieving the other plan objectives, in particular the dose distribution. For example, the plan objectives may comprise a maximum field delivery time for the hybrid radiation treatment. By defining a maximum field delivery time, the minimization of the plan cost value will take the delivery time into account. This may for example result in a greater portion of the radiation fields being transmission radiation fields.

The method further comprises calculating at least one plan cost value of the hybrid radiation treatment plan, the plan cost value being a measure of how well the plan objectives are being fulfilled by the hybrid radiation treatment plan. This calculation may involve the calculation of the dose distribution based on the current definition of the radiation fields. Each field produces its own dose contribution, and the contribution from all the fields together forms the plan dose, which is a primary plan objective. The calculation may for example be done by simulating the passage of each individual proton beam through the patient anatomy, as is known in the art. This simulation will enable to calculate the dose distribution and optionally further parameters of the treatment plan, and compare those to the plan objectives. The plan cost value depends on how well the plan objectives are met.

In the final step, the plan cost value is minimized by adjusting the radiation fields as received in step (i), thus the at least one conventional radiation field and/or the at least one transmission radiation fields. The adjustment may in particular involve changing the parameters of the radiation fields, for example the intensity (also referred to as "weight") of the individual proton beams or radiation fields, the gantry rotation angle, the number of beams, the number of energy layers, the energy of each beam and/or the geometry of the radiation fields. The intensity of a proton beam defines how much dose each beam ends up delivering, while the energy defines how deep in the patient the dose is deposited. It some embodiments, the range of energies is pre-selected to cover the target, so that the optimizer is not configured to adjust the energies of the individual proton beams or radiation fields.

The adjustment may further comprise adjusting the number of conventional fields and transmission fields. It may further comprise adjusting the gantry angle of each radiation field. This optimization process is generally known in the art, and is known as "inverse planning". In an embodiment, the minimization may be done in an iterative way. In particular, the minimization may be performed by an optimizer which iteratively adjusts the individual radiation fields and then goes back to step (iii), wherein the resulting dose distribution is again simulated and the plan cost value is calculated. Thus, based on the current plan cost value, the optimizer will adjust the individual proton beams within the radiation fields so that the doses that they produce better conforms to the objectives, thus reducing the overall cost. This and the previous step are repeated in an iterative manner until the optimizer is no longer able to make meaningful changes to the fields, at which point the process is said to have converged.

This process is generally known for the overall objectives, here referred to as "plan objectives". According to an embodiment of the invention, the optimizer includes the possibility of defining objectives for any group of radiation fields, for example the group comprising all transmission fields and the group comprising all conventional fields.

According to this embodiment, step (ii) comprises receiving one or more group objectives relating to at least one group of radiation fields, in particular to a group consisting of the conventional radiation fields and/or to a group consisting of the transmission radiation fields; (iii) comprises calculating group cost values for each of the groups of radiation fields; and (iv) comprises minimizing at least one group cost value by adjusting the fields in the respective group of radiation fields.

Thus, it possible for a user to define separate objectives for different groups of radiation fields. Generally, the radiation fields may be grouped together in any way. However, it appears preferable to group together all conventional radiation fields and all transmission radiation fields. Similarly, the optimizer is configured to calculate a cost value, referred to as "group cost value" for each group of radiation fields separately. In particular, the dose from each group may be observed separately. The optimizer is configured to minimize the group cost values by adjusting the fields in their respective group of radiation fields. Thereby, the treatment planner is able to purposefully use the advantages of the transmission radiation fields. For example, a group objective may be that the group of transmission radiation fields shall contribute more than a certain percentage to the overall dose to the target volume. The percentage may for example be between 20% and 80%, more preferred between 30% and 70%, more preferred between 40% and 60%, depending on the anatomy. A group objective may also be that the group should contribute a dose within the specified percentage ranges to the overall dose. Further group objectives may include specific directions or gantry angles from which a larger percentage of the overall dose should be delivered, for example because of concerns over patient movement or inhomogeneities along the field path.

According to an embodiment, step (ii) comprises receiving one or more field objectives relating to at least one individual radiation field; step (iii) comprises calculating field cost values for the at least one individual radiation field; and step (iv) comprises minimizing at least one field cost value by adjusting the at least one individual radiation field.

Thus, the objectives may also be given for individual radiation fields. These objectives may again be defined by a user or treatment planner, same as the group objectives. For example, the user may define a planned dose for a specific radiation field. Field objectives and group objectives may also be derived from a database containing example treatment plans which have worked well in the past for the respective type of patient anatomy and target volume. The field objectives can also be unrelated to dose, for example they may include minimum and/or maximum values for the beam intensities in the radiation field. This is because one often wants to avoid beams with very low or very high intensity. In this embodiment, the optimizer is configured to calculate field cost values for those individual radiation fields for which a field objective is defined, and minimizing the respective field cost value by adjusting the respective individual radiation field. The adjustment comprises adjusting parameters of the respective radiation field, for example the energy of the individual proton beams (in particular for Bragg peak beams), the gantry rotation angle, the number of beams, and/or the geometry of the radiation field.

It is possible for a user to specify group objectives and/or individual field objectives for all groups and fields, or only for some of them.

Thus, in an embodiment the optimization algorithm is capable of handling hybrid radiation treatment plans by minimizing the cost values not only of the overall treatment plan, but also of specific groups of radiation fields and even individual radiation fields. For example, a field objective may comprise a minimum dose to be delivered by the specific radiation field.

According to an embodiment, a user may give different priorities to the different objectives. In particular, the group objectives and the field objectives, where given, may include a priority value. The priority value will be taken into account in the minimization step of the respective cost value, so that the objectives with higher priority values will be achieved with higher priority.

The invention also relates to a control feedback system for defining and reviewing the optimization objectives for the plan, individual radiation fields, and groups of radiation fields. Preferably, the control feedback system offers a simultaneous view and control not only for the overall treatment plan, but also for individual fields and field groups in a self-contained optimization dialog.

According to an embodiment, the method comprises providing a user interface, the user interface
- allowing a user to define at least one conventional radiation field and/or at least one transmission radiation field as well as at least one associated field objective, the field objective comprising at least a minimum dose to be delivered to the target volume or a part of the target volume, wherein the definition optionally comprises a gantry rotation angle,
- providing a visualization of the dose distribution delivered by the hybrid treatment plan, wherein the visualization optionally comprises a Dose Volume Histogram.

The control feedback system preferably makes use of a user interface allowing a user to define the radiation fields as well the associated fields objectives, and providing a visualization of the dose distribution delivered by the hybrid treatment plan. The visualization may for example comprise a visualization of the 3D image forming the basis for the treatment plan, with the dose distribution overlaid thereon. The visualization may additionally comprise a dose volume histogram (DVH), which may include a DVH for the target volume and for one or more organs at risk. Thus, the user interface preferably allows the treatment planner to define the objectives for each field individually, and to view the planning result, i.e. the dose distribution of the hybrid treatment plan. Preferably, there are separate controls for defining which radiation fields or groups of fields the field objectives or group objectives are associated with. This makes it fast and intuitive to set objectives e.g. for the entire treatment plan, for individual conventional/transmission fields, or for groups of fields.

In an embodiment, the user interface allows a user to select one or more or all of the radiation fields, and wherein the visualization relates to the selected radiation fields, and in particular visualizes the summed-up dose distribution of the selected radiation fields.

Thereby, the controlled feedback system allows the treatment planner to observe the dose distribution not only from the overall hybrid treatment plan, but for individual fields or individual field groups. Thereby, the treatment planner is effectively assisted in defining suitable transmission or conventional fields.

According to an embodiment, the user interface comprises an input field allowing a user to define objectives for a single radiation field or for an arbitrarily selectable group of radiation fields. Thus, the controlled feedback system allows the treatment planner to define objectives for any combination of transmission and conventional radiation fields. This is highly advantageous for being able to generate high quality plans containing transmission beams. In particular, the user interface allows the treatment planner to define the rotation angle for each transmission and radiation field. In other words, the system allows the treatment planner to define any combination of transmission and Bragg peak beams with any combination of beam angles.

According to an embodiment, the adjusting the at least one conventional radiation field and/or the at least one transmission radiation field comprises adjusting at least one of
- proton beam energies,
- number of proton beams, and
- gantry rotation angle.

The optimization of radiation fields in the context of proton treatment planning is generally known. It is an iterative process wherein the parameters of the radiation fields are adjusted in order to minimize the cost values, be it the field cost values, group cost values or plan cost values. The parameters that can be adjusted may be at least one of the proton beams energies, for example the energies and number of layers of the pencil beams in a particular radiation field. Also, the number of pencil beams within one radiation field may be adjusted. Further, the geometry of each radiation field may of course be adjusted to the size of the target volume. Finally, the gantry rotation angle may be varied. In an embodiment, the minimalization step may also adjust the number of radiation fields. These adjustments may be done to each conventional radiation fields and transmission radio field.

According to an embodiment, the patient anatomy is the patient's breast and the hybrid radiation treatment plan comprises at least one tangential transmission radiation field.

It has been shown that the inventive hybrid radiation treatment plan is especially advantageous in the treatment of breast cancer. This is because even after accelerating the delivery time of the (conventional) IMPT fields with the available optimization techniques, the delivery can take multiple minutes per field, in particular if full breast irradiation is required. With the inventive hybrid radiation treatment plans, at least one of the conventional radiation fields may be replaced by a tangential transmission field, which drastically reduces the field delivery time without affecting the dose to normal tissue. By "tangential" positioning of the transmission radiation field, it is meant that the beams cross the breast tissue tangentially to the ribcage. Therefore, the radiation of the transmission beams does not pass through any organs at risk. For example, the radiation treatment plan comprises 1 or 2 tangential transmission radiation fields, and 1 to 3, preferably 1 to 2, conventional radiation fields.

According to another embodiment, the target volume is a head and neck cancer and the hybrid radiation treatment plan comprises a plurality of transmission radiation fields and up to two conventional radiation fields, preferably one conventional radiation field.

A further advantageous application of the invention is head and neck cancer (HNC). In a typical HNC case, three conventional radiation fields are used. By replacing two of the conventional fields with transmission fields, it is possible to drastically cut the total delivery time, while the optimizer may still ensure that the dose to OAR is only slightly affected. Thus, in a preferred embodiment, a hybrid treatment plan may comprise a plurality, for example 1 to 3, more preferred 1 to 2, transmission radiation fields, and up to 2 conventional radiation fields, preferably one conventional radiation field. While the hybrid treatment plan yields slightly worse dose distribution for some of the organs at risk, the plans still have significantly higher delivery efficiency thanks to all transmission fields being delivered in under 5 seconds.

Preferably, a transmission radiation field may be delivered to the patient in less than 5 seconds, preferably in 1 to 4 seconds.

In another aspect, the invention relates to a computer program or digital storage medium comprising instructions which will cause a processing unit to perform the method according to any one of the preceding claims, when the computer program is being executed on the processing unit. The computer program may be part of a proton treatment planning software. It may be executed on any kind of processing unit, for example on a CPU or GPU, cloud computer or mobile device. Preferably, the processing unit is connected to a suitable user interface in order to allow the treatment planner to define the different radiation fields. Further, the computer program may comprise an optimizer for minimizing the various cost values, wherein the optimization step may rely on known optimization algorithms such as available in MatRad. The digital storage medium may be any suitable data storage medium, for example an optical, magnetic or solid-state storage medium. It may for example be a cloud computer, a hard disk, a server, an SSD card, an SD card, a CD-ROM, or a laptop or mobile device.

According to a further aspect, the invention relates to a system for generating a hybrid radiation treatment plan for proton therapy of a patient, the system comprising:
- an interface configured to receive a patient anatomy including a target volume, the target volume to be treated with a proton therapy, and to provide a hybrid radiation treatment plan comprising at least one conventional radiation field and a least one transmission radiation field;
- a calculation unit configured for defining at least one conventional radiation field comprising one or more Bragg peak proton beams, each Bragg peak proton beam having a Bragg peak within the target volume and at least one transmission radiation field consisting of one or more transmission proton beams, each transmission proton beam having sufficiently high energy to reach a distal exit side of the patient.

In particular, the system may be a computer, cloud computer, mobile device or proton treatment planning system. It comprises an interface for receiving various input data, in particular a 3D image of the patient anatomy and/or delineations of the target volume. It may also have an interface for outputting the hybrid proton radiation treatment plan. It may further comprise a display for showing the calculated dose distribution and DVHs to the user. The system further comprises a calculation unit for performing the method steps described herein. The calculation unit may be a processing unit as described above. In particular, it may be configured to execute the method described herein.

All embodiments and advantages described with respect to the method also applies to the computer program, the digital storage medium, the system for generating the treatment plan and the proton therapy system, and vice versa.

According to a further aspect, the invention relates to a proton therapy system configured to generate one or more proton fields in order to expose a target volume within a patient body to proton radiation, wherein the proton therapy system is configured to perform a method comprising:
- delivering at least one conventional radiation field comprising one or more Bragg peak proton beams, each Bragg peak proton beam having a Bragg peak within the target volume;
- delivering at least one transmission radiation field consisting of one or more transmission proton beams, each transmission proton beam having sufficiently high energy to reach a distal exit side of the patient.

The proton therapy system preferably comprises a gantry and other components of a proton therapy system, in particular a cyclotron, a patient couch, an imaging system etc. The system is preferably configured to execute the method described herein.

The invention shall now be described by means of embodiments with references to the attached drawings. In the drawings:
- Fig. 1: shows an illustration of a conventional radiation treatment plan with two conventional IMPT fields (A), a hybrid radiation treatment plan with one conventional field and one transmission field (B) and of a plan with one conventional IMPT field and one combined conventional+transmission field (C);
- Fig. 2: shows uncertainty DVHs of the example plans of Fig. 1.
- Fig. 3: shows a user interface according to an embodiment of the invention;
- Fig. 4: shows a flow diagram of a minimization algorithm;
- Fig. 5: schematically shows an axial slice through a breast cancer patient illustrating a hybrid treatment plan;
- Fig. 6: shows a schematic representation of a proton therapy system.

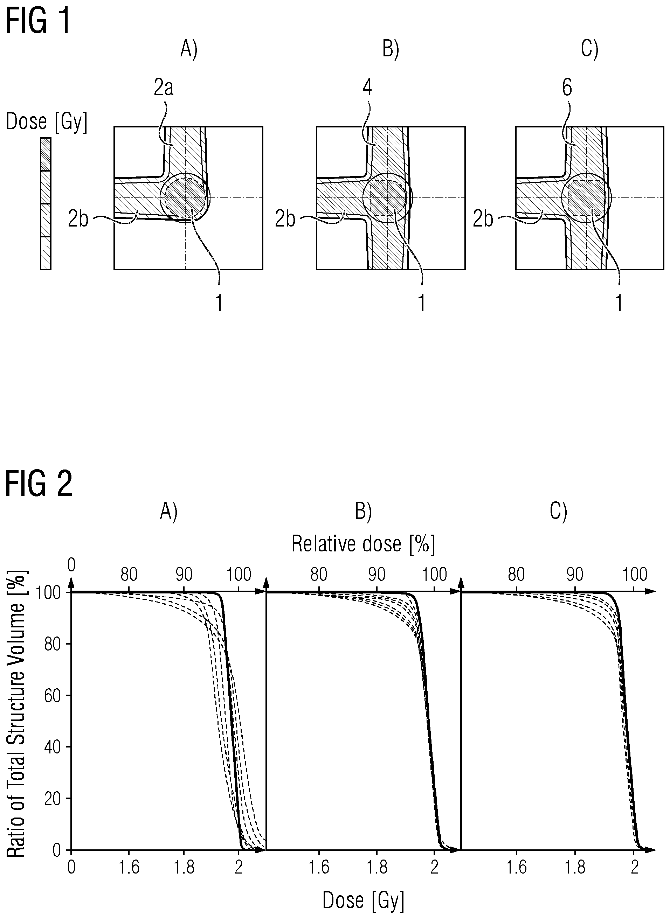

Fig. 1 is a schematic representation of three different treatment plans. The left section A shows an example traditional treatment plan comprising two conventional radiation fields 2a, 2b. The target volume 1 is depicted in the middle, and it can be seen that the conventional fields 2a and 2b do not extend behind the target volume 1, in other words they have Bragg peaks within the target volume 1. In this example, the depicted fields are IMPT fields, having multiple energy layers. The middle section B, by contrast, shows a hybrid plan with one conventional IMPT field 2b and one transmission field 4. It can be seen that the transmission field 4 has steep fall-offs at the side, but extends through the target volume 1, exiting the field of view on the distal side of the target volume 1. In other words, the exit dose from the transmission field 4 is clearly visible. Section C shows a combined treatment plan, having one conventional field 2b and one combined field 6 having both conventional and transmission beams. For convenience, conventional fields will in the following be identified with numbers (1, 2, 3,...) and transmission fields with alphabets (A, B, C,...).

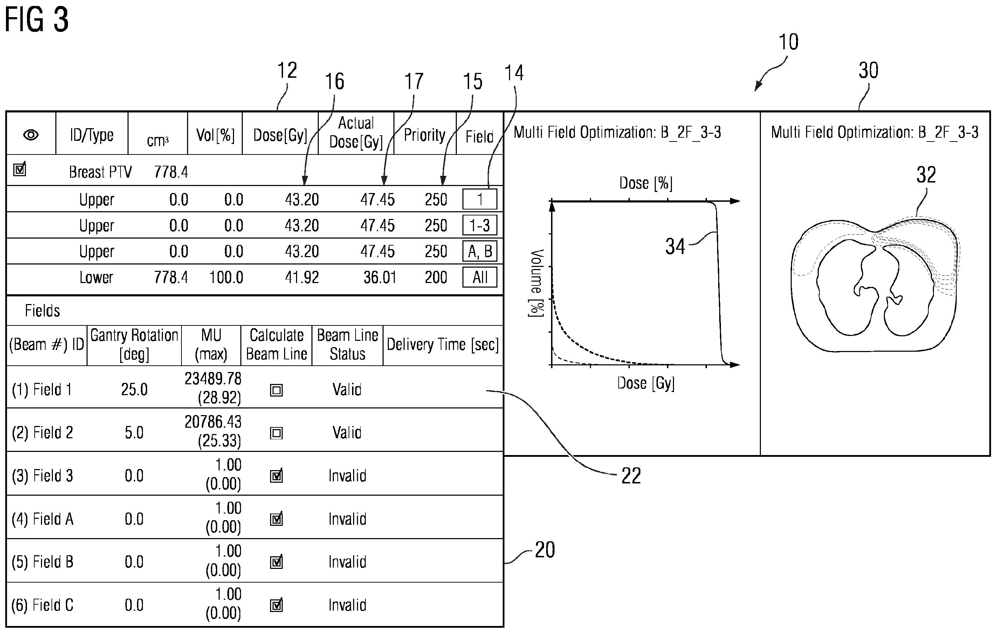

The calculated field delivery times for the treatment plans of Fig. 1 is shown in table 1 below. Since the transmission fields only take one second to deliver, the hybrid plan of Fig. 2 takes much less time to deliver than the plans of both Fig. 1 and 3.

**Table 1: Field delivery times for treatment plans A)-C) in Fig. 1.**

| Plan | Delivery Time | | |
|---|---|---|---|
| | Conventional/ Combined | Transmission | Total |
| A) | 17+ 19s | 0 s | 36 s |
| B) | 19 s | 1 s | 20 s |
| C) | 14 + 18s | 1 s | 33 s |

Fig. 2 further illustrates the uncertainty dose volume histograms of the three example plans A, B, C of Fig. 1. The robustness of the plans is shown in dotted lines. Thus, one can see that the robustness is superior in the plans with transmission field (B) and combined field (C), even without explicit robust optimization. The nominal dose of each plan is nearly identical.

Fig. 3 illustrates the user interface of an embodiment of the proposed control feedback system. In the top left area A/12, the objectives of each radiation field and radiation field group may be entered by a user. Each line represents one field or a arbitrarily selected group of fields. In the boxes 14, the target fields may be indicated in any combination in order to identify which field or fields each objective is associated with. The other numbers and boxes indicate typical objectives, such as priority 15, dose 16 or actual dose 17. The priority values 15 are taken into account when calculating the costs values, so that the objectives may be weighted by adjusting the priorities.

In the area 20 on the bottom left, the various fields, in this case conventional fields 1-3 and transmission fields A-C are listed, each in one line 22. By clicking/selecting one or several lines in this area 20, a user may select which fields are to be visualized in the region 30 on the right of the screen. In the present case, field A is selected, and accordingly the dose distribution 32 and DVH 34 of this field is illustrated in the display region 30. In case all fields 22 are selected in area 20, the dose shown in 30 is the plan dose. Thereby, the treatment planner is given a simultaneous view and control not only of the overall treatment plan, but also for individual fields and field groups in an idividual, self-contained optimization dialog. According to this embodiment, the user has separate controls for defining which fields or group of fields the objectives are associated with, through the selection boxes 14 in region 12. The method is fast enough to simulate the associated dose distributions and DVHs - preferably at first without any optimization. Rather, the user is given immediate feedback, since the treatment planner may observe the dose from individual fields or field groups. This makes it fast and intuitive to set objectives e.g. for the entire treatment plan, for individual conventional fields, individual transmission fields, or for a group of fields, for example the group comprising all conventional fields and / or all transmission fields.

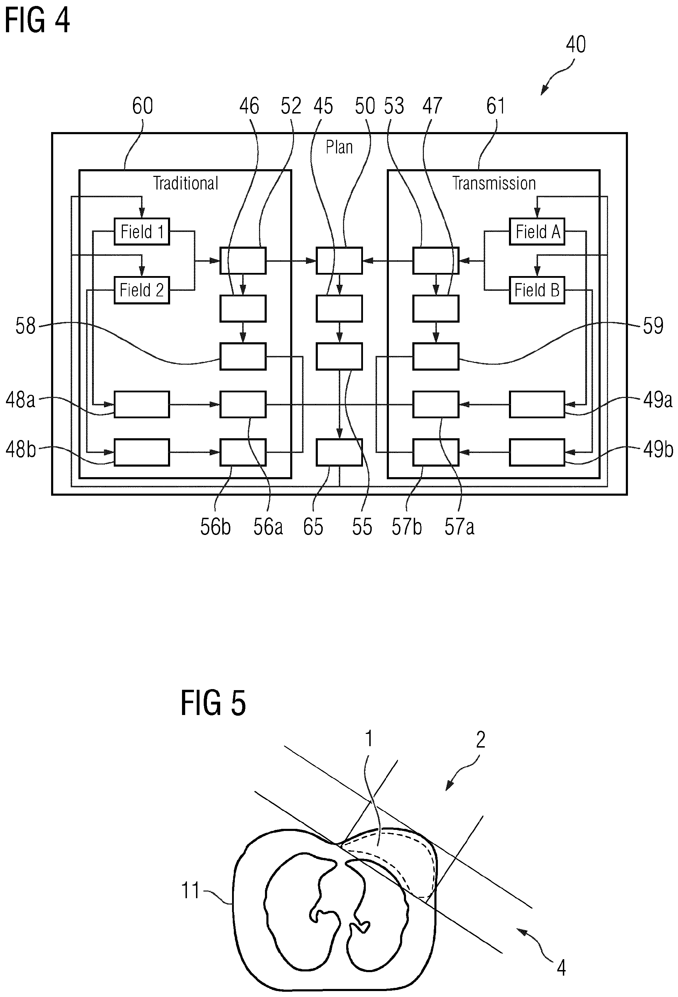

Fig. 4 illustrates the data flow in the optimization algorithm. The algorithm receives the relevant patient data (not shown), the defined conventional fields 1, 2 and transmission fields A, B, and the optimization objectives, in particular the field objectives 48a und 48b of conventional fields 1 and 2, as well as the field objectives 49a, 49b of the fields A and B to the algorithm. Note that the data flow is divided into a part relating to the overall hybrid plan in the centre, in a part 60 for the optimization of the conventional fields 1, 2, and in a part 61 on the right for the optimization of the transmission fields A, B. It should be noted that the same approach can be used for any other combination of the fields, since the groups of fields may be arbitrarily selected. Looking at the data flow 60, the algorithm will first calculate the traditional/convnetionaldose 52 from the two conventional fields 1, 2. From the available data and the received objectives 46 for the conventional fields together, it will calculate the group cost value 58 of the group of conventional fields. In a parallel calculation, the algorithm will calculate the field cost value 56a from the objectives of the first conventional field 48a and the field cost value 56b from the objectives of the second conventional field 48a. Thus, cost values are now available field-by-field, as well as for each group of fields.

The same is done for the transmission fields in the data flow 61: from the definition of fields A and B, the algorithm will calculate the transmission dose 53. From the received objective 47 for the group of transmission fields, the group cost value 59 of the group of transmission fields will be computed. In parallel, the algorithm will also calculate the field cost values 57a and 57b from the field objectives 49a, 49b defined for the transmission fields A, B. These cost values may be combined to the total cost 65. The total cost may also be compared to the plan cost 55 resulting from the plan objectives 45 and the plan dose 50. Once the total cost 65 has been computed, the fields 42a, 42b as well as 43a, 43b are adjusted to find an optimum. In an embodiment, the cost values are calculated based on the priority 15 given to each objective.

Fig. 5 schematically shows an axial view through a breast cancer patient, to illustrate the use of hybrid treatment plans for breast cancer. The objectives include providing left side full breast irradiation without the inclusion of notes. It has been found that, even after accelerating the delivery time of the IMPT fields by available optimisation techniques, the delivery may take multiple minutes per field (see below table 2). Using a hybrid plan B with two conventional and one transmission fields, or a hybrid plan C with one transmission field 4 and one conventional field 2, the field delivery time is drastically reduced without affecting the dose to normal tissue, thanks to the tangential positioning of the transition field 6. Both conventional and hybrid plans have virtually identical to those statistics, even though the field delivery time of the hybrid plans are reduced by about 40%.

**Table 2: Field delivery times of treatment plan in Figure 5.**

| Plan | Delivery Time | | |
|---|---|---|---|
| | Conventional | Transmission | Total |
| A) | 144+125 s | 0 s | 269 s |
| B) | 80+73 s | 8s | 161 s |
| C) (see Fig. 5) | 145 s | 7s | 152 s |
| 1 Traditional + 2 Transmission beams | 99s | 6+7 s | 112 s |

It has been found that the hybrid radiation treatment plans can achieve competing low organ at risk doses compared to conventional Bragg peak IMPT plans, but with much faster delivery, for at least head and neck cancer and breast cancer plans.

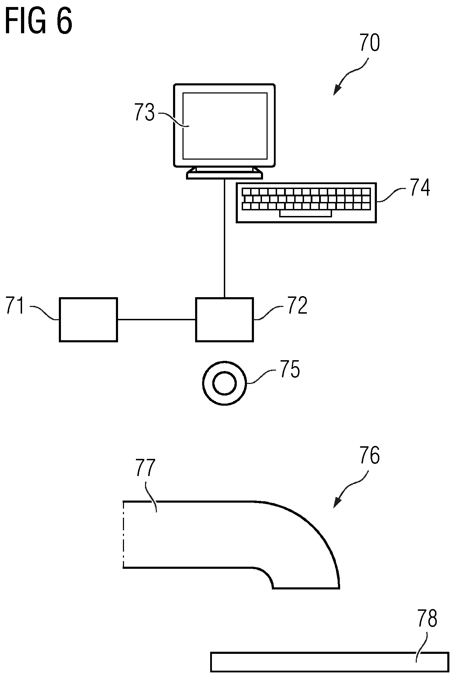

Fig. 6 schematically shows a system 70 for generating the hybrid radiation treatment plan, as well as the proton therapy system 76. The system 70 for generating the treatment plan includes a calculation unit 72, and an interface 71 to a database for receiving the patient anatomy. The user may interact with the system via user interface 74, for example a keyboard. The dose distribution may be viewed on a screen 73. The radiation treatment system 76 includes a gantry 77 for delivering proton beams at defined energies and rotation and further equipment which is not shown. The patient may be placed on a patient couch 78.

### List of Reference Signs

- 1: target volume
- 2: conventional radiation field
- 4: transmission radiation field
- 6: combined radiation field
- 10: user interface
- 11: patient anatomy
- 12: field objective definition area
- 14: field selection box
- 15: priority box
- 16: dose box
- 17: actual dose box
- 20: field selection area
- 22: field line
- 25: gantry rotation
- 30: visualisation area
- 32: dose distribution visualisation
- 34: dose volume histogram of target
- 35: dose volume histogram of OAR
- 40: process flow diagram
- 45: plan objectives
- 46: traditional objectives
- 47: transmission objectives
- 48a: field objectives for conventional field 1
- 48b: field objectives for conventional field 2
- 49a: field objectives for transmission field A
- 49b: field objectives for transmission field B
- 50: plan dose
- 52: traditional dose
- 53: transmission dose
- 55: plan cost
- 56a: field 1 cost
- 56b: field 2 cost
- 57a: field A cost
- 57b: field B cost
- 58: traditional cost
- 59: transmission cost
- 60: optimisation of traditional feels
- 61: optimisation of Transmission Fields
- 65: total cost
- 70: system for generating a hybrid treatment plan
- 71: interface
- 72: calculation unit
- 73: screen
- 74: user input device
- 75: proton therapy system
- 77: Gantry
- 78: patient couch

## Claims

1. A computer-implemented method for generating a hybrid radiation treatment plan for proton therapy of a patient, the method comprising:
(a) receiving a patient anatomy including a target volume (1), the target volume to be treated with a proton therapy;
(b) defining at least one conventional radiation field (2) comprising one or more Bragg peak proton beams, each Bragg peak proton beam having a Bragg peak within the target volume;
(c) defining at least one transmission radiation field (4) consisting of one or more transmission proton beams, each transmission proton beam having sufficiently high energy to reach a distal exit side of the patient; and
(d) providing a hybrid radiation treatment plan comprising the at least one conventional radiation field (2) and the a least one transmission radiation field (4).

2. The method of claim 1, wherein at least one of the at least one conventional radiation fields (2) is a combined radiation field (6) comprising one or more Bragg peak proton beams and one or more transmission proton beams.

3. The method of claim 1 or 2, comprising the steps of
(i) receiving a definition of at least one conventional radiation field (2) and of at least one transmission radiation field (4);
(ii) receiving one or more plan objectives (45) for the hybrid radiation treatment plan, the plan objectives (45) comprising at least a minimum dose to be delivered within the target volume and optionally a maximum dose to be delivered to an organ at risk within the patient anatomy;
(iii) calculating at least one plan cost value (55) of the hybrid radiation treatment plan, wherein the at least one plan cost value is a measure of how well the plan objectives (45) are being fulfilled by the hybrid radiation treatment plan; and
(iv) minimizing the at least one plan cost value by adjusting the at least one conventional radiation field (2) and/or the at least one transmission radiation field (4).

4. The method of claim 3,
- wherein step (ii) comprises receiving one or more group objectives (46, 47) relating to at least one group of radiation fields, in particular to a group consisting of the conventional radiation fields (2) and/or to a group consisting of the transmission radiation fields (4);
- wherein step (iii) comprises calculating group cost values (58, 59) for each of the groups of radiation fields; and
- wherein step (iv) comprises minimizing at least one group cost value (58, 59) by adjusting the fields (1, 2, A, B) in the respective group of radiation fields.

5. The method of claim 3 or 4,
- wherein step (ii) comprises receiving one or more field objectives (48a, 48b, 49a, 49b) relating to at least one individual radiation field (1, 2, A, B);
- wherein step (iii) comprises calculating field cost values (56a, 56b, 57a, 57b) for the at least one individual radiation field (1, 2, A, B); and
- wherein step (iv) comprises minimizing at least one field cost value (56a, 56b, 57a, 57b) by adjusting the at least one individual radiation field (1, 2, A, B).

6. The method of any one of claims 3 to 5, wherein the plan objectives comprise a maximum field delivery time for the hybrid radiation treatment.

7. The method of any one of the preceding claims, comprising providing a user interface (10) , the user interface
- allowing a user to define at least one conventional radiation field (2) and/or at least one transmission radiation field (4) as well as at least one associated field objective, the field objective comprising at least a minimum dose (12) to be delivered to the target volume (1) or a part of the target volume, wherein the definition optionally comprises a gantry rotation angle,
- providing a visualization of the dose distribution (32) delivered by the hybrid treatment plan, wherein the visualization optionally comprises a Dose Volume Histogram (34).

8. The method of claim 7, wherein the user interface (10) allows a user to select one or more or all of the radiation fields (14), and wherein the visualization relates to the selected radiation fields (14) , and in particular visualizes the summed-up dose distribution of the selected radiation fields.

9. The method of claim 7 or 8, wherein the user interface comprises an input field (12) allowing a user to define objectives for a single radiation field or for an arbitrarily selectable group of radiation fields.

10. The method of any one of claims 3 to 9, wherein the adjusting the at least one conventional radiation field (2) and/or the at least one transmission radiation field (4) comprises adjusting at least one of
- proton beam weights,
- number of proton beams, and
- gantry rotation angle.

11. The method of any one of the preceding claims, wherein the patient anatomy is the patient's breast and the hybrid radiation treatment plan comprises at least one tangential transmission radiation field (4).

12. The method of any one of the preceding claims, wherein the target volume is a head-and-neck cancer and the hybrid radiation treatment plan comprises a plurality of transmission radiation fields (4) and up to two conventional radiation fields (2), preferably one conventional radiation field (2).

13. Computer program or digital storage medium (75) comprising instructions which will cause a processing unit (72) to perform the method according to any one of the preceding claims, when the computer program is being executed on the processing unit.

14. A system ((70) for generating a hybrid radiation treatment plan for proton therapy of a patient, the system comprising:
- an interface (71) configured to receive a patient anatomy including a target volume, the target volume to be treated with a proton therapy, and to provide a hybrid radiation treatment plan comprising at least one conventional radiation field (2) and a least one transmission radiation field (4);
- a calculation unit (72) configured for defining at least one conventional radiation field (2) comprising one or more Bragg peak proton beams, each Bragg peak proton beam having a Bragg peak within the target volume and at least one transmission radiation field (4) consisting of one or more transmission proton beams, each transmission proton beam having sufficiently high energy to reach a distal exit side of the patient.

15. A proton therapy system (76) configured to generate one or more proton fields in order to expose a target volume within a patient body to proton radiation, wherein the proton therapy system is configured to perform a method comprising:
- delivering at least one conventional radiation field (2) comprising one or more Bragg peak proton beams, each Bragg peak proton beam having a Bragg peak within the target volume;
- delivering at least one transmission radiation field (4) consisting of one or more transmission proton beams, each transmission proton beam having sufficiently high energy to reach a distal exit side of the patient.
